**Europäisches Patentamt**

**European Patent Office**

**Office européen des brevets**

(19)

(11) Veröffentlichungsnummer: **0 403 683**

**A1**

## (12) EUROPÄISCHE PATENTANMELDUNG

(21) Anmeldenummer: **89111499.3**

(22) Anmeldetag: **23.06.89**

(51) Int. Cl.⁵: **A61B 5/00**

(43) Veröffentlichungstag der Anmeldung:
**27.12.90 Patentblatt 90/52**

(84) Benannte Vertragsstaaten:
**BE DE ES FR GB NL SE**

(71) Anmelder: **Siemens Aktiengesellschaft**
**Wittelsbacherplatz 2**
**D-8000 München 2(DE)**

(72) Erfinder: **Schelter, Wolfgang, Dr.**
**Eichendorffstrasse 7**
**D-8525 Uttenreuth(DE)**
Erfinder: **Gumbrecht, Walter, Dr.**
**In der Röte 1**
**D-8522 Herzogenaurach(DE)**
Erfinder: **Montag, Bernhard**
**Nordring 22**
**D-8550 Forchheim(DE)**

(54) **Anordnung zur Untersuchung eines flüssigen Messmediums.**

(57) Zur Bestimmung von Blutgasen ist eine optische Meßzelle, die das Blut enthält, und eine Elektronik zur Verarbeitung des Ausgangssignals vorgesehen. Erfindungsgemäß enthält der Meßkanal (12) in der Meßzelle (10) strömendes Blut. Der Meßkanal (12) verbindet eine Vorrichtung (28) zur Konstanthaltung der Strömungsgeschwindigkeit im Meßkanal (12) derart über einen Doppellumenkatheter (3) sowohl mit dem Meßmedium (9) als auch mit einer Infusionslösung (39), daß der Meßzelle (10) jeweils abwechselnd das Meßmedium (9) und die Infusionslösung (39) zuführbar ist. Mit dieser Vorrichtung kann zugleich die Sauerstoffsättigung SaO₂ und der Hämoglobingehalt Hb des Blutes und mit einer dritten Wellenlänge auch die Konzentration eines Indikatorfarbstoffes bestimmt werden.

FIG. 2

Xerox Copy Centre

## Anordnung zur Untersuchung eines flüssigen Meßmediums

Die Erfindung bezieht sich auf eine Anordnung zur Untersuchung eines flüssigen Meßmediums, vorzugsweise zur Bestimmung von Blutgasen, insbesondere der Sauerstoffsättigung $SaO_2$ und des Hämoglobingehalts Hb sowie der Konzentration eines Indikatorfarbstoffes im Vollblut. Die Anordnung enthält eine Meßzelle, die das Meßmedium enthält. Dieser Meßzelle sind eine Strahlungsquelle und ein Strahlungsempfänger zugeordnet, dessen Ausgangssignal in einer Elektronik verarbeitet wird.

Transmissionsuntersuchungen von Vollblutproben mit rotem und infrarotem Licht zeigen, daß die Intensitätsänderung der Strahlung zur Bestimmung der Sauerstoffsättigung und des Hämoglobingehalts des Blutes herangezogen werden kann.

Eine bekannte Ausführungsform einer Anordnung zur Bestimmung der Sauerstoffsättigung $SaO_2$ enthält zwei Leuchtdioden, nämlich für eine Rotstrahlung bei einer Wellenlänge von etwa 660 nm und einer Infrarotstrahlung bei einer Wellenlänge von 950 nm, deren Strahlung eine Blutprobe durchsetzt und einem gemeinsamen Strahlungsempfänger zugeführt wird, der beispielsweise eine Photodiode sein kann. Die Transmissionssignale können je nach der Ansteuerung der Lichtquellen einzeln oder additiv gemessen werden. Das Blut mit einem Volumen von beispielsweise etwa 20 $\mu$l befindet sich in einer Glaskapillare. Die Blutproben können vor der Messung hämolysiert werden. Die Ausgangssignale werden über einen Analog-Digital-Wandler einem Rechner zugeführt. Mit Hilfe einer Eichkurve, die aus einem größeren Kollektiv von Blutproben empirisch bestimmt wird, kann dieser Verrechnungswert in einen Sauerstoffsättigungswert umgesetzt und angezeigt werden. Man erhält somit ein automatisches Meßgerät, das jedoch vor jeder Messung geeicht werden muß (Biomedizinische Technik, Bd. 30, Heft 1-2/1985, Seiten 18 bis 23).

Bei der sogenannten Puls-Oximetrie wird ein Körperglied, beispielsweise eine Fingerspitze, zwischen einer Strahlungsquelle mit zwei Wellenlängen und einem Strahlungsdetektor angeordnet. Bei jedem Herzschlag weitet sich die Arterie etwas aus und der optische Weg der Durchstrahlung wird entsprechend geändert. Als Strahlungsquellen sind im allgemeinen zwei Leuchtdioden für die verschiedenen Wellenlängen vorgesehen. Ein gemeinsamer Strahlungsempfänger zeigt die Intensitätsänderung der Strahlung an. Die Strahlung einer Wellenlänge bestimmt die Absorptionsänderung, während die zweite Wellenlänge zur Bestimmung der Änderung des optischen Weges herangezogen wird. Diese Pulsoximeter stellen zwar eine besonders einfache Anordnung zur Bestimmung von Blutgasen dar, sie liefern jedoch verhältnismäßig ungenaue Ergebnisse (Journal of Physics E: Sci. Instr., Vol. 20, No. 9 (1987), Seiten 1103 bis 1112, insbesondere Figur 19).

Zur direkten Bestimmung der Sauerstoffsättigung und der Farbstoffkonzentration im Vollblut kann auch eine Licht-Reflexionssonde vorgesehen sein, die mit Lichtleitern als optischer Koppler versehen ist. Dieser Fieberoptikkatheter verbindet eine Strahlungsquelle für drei verschiedene Wellenlängen mit einem Analysator, der das im Blutstrom reflektierte und verstärkte Signal verarbeitet. Der Analysator enthält eine elektronische Weiche für die Signale der verschiedenen Wellenlängen sowie einen Dividierer und eine Digitalanzeige. Mit dieser Anordnung kann kontinuierlich der im strömenden Blut gebundene Sauerstoff festgestellt werden. Die Messung der Konzentration von stromaufwärts injiziertem Farbstoff kann beispielsweise zur Leberfunktionsprüfung verwendet werden. Der Meßvorgang erfolgt an der Katheterspitze und damit unmittelbar an jeder Stelle der Blutkreislaufs, die mit dem Katheter erreichbar ist. Mit dieser Einrichtung können die Meßwerte digital angezeigt und als analoges elektrisches Signal ausgegeben werden. Die Anordnung muß jedoch vor jeder Messung geeicht werden (Medizintechnik, 107. Jahrgang, 3/87, Seiten 107 und 108).

Eine weitere Ausführungsform einer Anordnung zur Untersuchung von Blut enthält einen Meßkanal, der mit einem Meßsensor und einem Referenzsensor versehen ist, und der mit dem inneren Lumen eines Doppellumenkatheters verbunden ist. Das äußere Lumen des Katheters wird mit Infusionslösung versorgt. Beide Lumen haben eine gemeinsame Mündung, die sich in dem strömenden Blut befindet. Durch wiederholte Umkehrung der Strömungsrichtungen in den Lumen mittels einer umsteuerbaren Pumpe wird eine Folge von abwechselnd- Meßmedium und Infusionslösung über zwei Sensoren gezogen, die im Meßkanal in einem vorbestimmten Abstand voneinander angeordnet sind (europäische Patentanmeldung 0 273 258).

Der Erfindung liegt die Aufgabe zugrunde, eine Anordnung zur Untersuchung eines flüssigen Meßmediums anzugeben, das eine kontinuierliche Messung erlaubt und eine Eichung nicht benötigt.

Diese Aufgabe wird erfindungsgemäß gelöst mit den kennzeichnenden Merkmalen des Anspruchs 1. Mit dieser Anordnung können sowohl Blutgase, insbesondere die Sauerstoffsättigung $SaO_2$ sowie der Homoglobingehalt Hb, als auch die Konzentration eines Indikatorfarbstoffes von Vollblut bestimmt werden. Mit diesen Gestaltungsmerkmalen erhält man eine Anordnung zur kontinuierlichen

Bestimmung der Sauerstoffsättigung $SaO_2$ und des Hämoglobingehalts Hb von Vollblut. In einer Ausführungsform mit einer Strahlungsquelle für drei verschiedene Wellenlängen kann auch die Konzentration eines Indikatorfarbstoffes in Vollblut bestimmt werden. Durch die abwechselnde Zufuhr von Vollblut und Infusionslösung ist diese Anordnung selbsteichend.

In einer besonders vorteilhaften Ausführungsform der Anordnung zur Untersuchung eines flüssigen Meßmediums können Strahlungsquelle und Strahlungsempfänger auf der gleichen Seite des Meßkanals angeordnet sein, insbesondere in einem gemeinsamen Halbleiterkörper integriert sein.

Zur optischen Kopplung können auch Lichtleiter vorgesehen sein, welche die Strahlung dem Meßmedium zuführen und vom Meßmedium wieder abführen.

In einer Ausführungsform mit einer Strahlungsquelle für drei verschiedene Wellenlängen kann die Anordnung auch zur Bestimmung der Konzentration eines Indikatorfarbstoffes im Meßmedium dienen.

Zur weiteren Erläuterung der Erfindung wird auf die Zeichnung Bezug genommen, in deren Figur 1 eine Anordnung zur Bestimmung von Blutgasen, insbesondere der Sauerstoffsättigung $SaO_2$, schematisch veranschaulicht. In Figur 2 ist die Anordnung der Meßzelle im Entnahmesystem für das Meßmedium dargestellt. Figur 3 zeigt eine besondere Ausführungsform der Meßzelle. Eine Ausführungsform mit Lichtleitern zur Strahlungsführung ist in Figur 4 veranschaulicht.

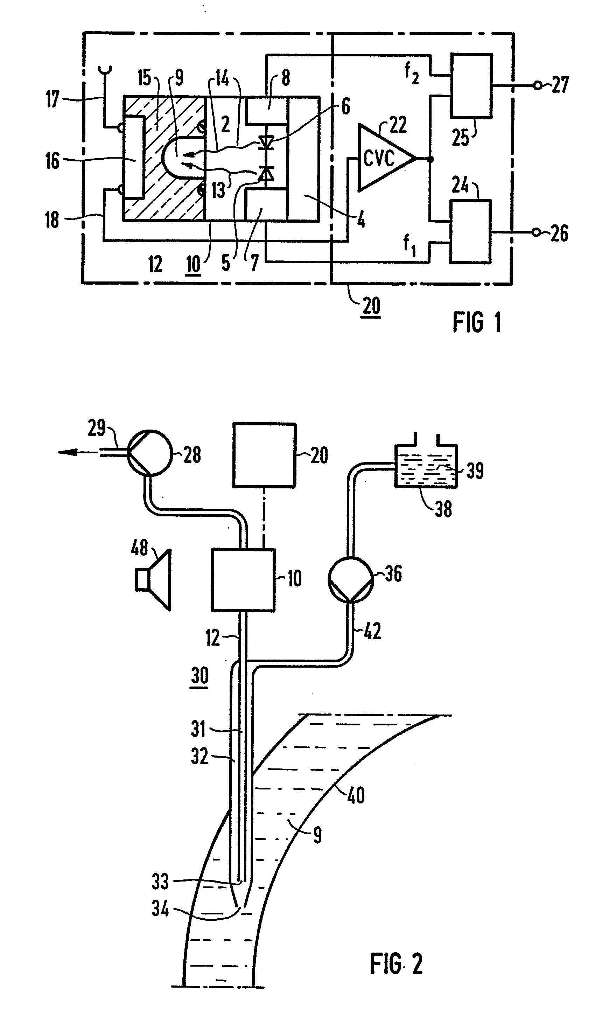

In der Ausführungsform einer Anordnung zur Untersuchung von Vollblut, insbesondere der Bestimmung der Sauerstoffsättigung $SaO_2$ sowie des Hämoglobingehalts Hb, gemäß Figur 1 ist eine Meßzelle 10 vorgesehen, der eine Elektronik 20 zugeordnet ist. Die Meßzelle 10 enthält eine Strahlungsquelle 2 für zwei Wellenlängen, vorzugsweise eine Leuchtdiode 5, beispielsweise für Rotstrahlung, mit einer Wellenlänge $\lambda_1$ = 660 nm und einer weiteren Leuchtdiode 6 für Infrarotstrahlung mit einer Wellenlänge $\lambda_2$ = 950 nm. Die zugehörigen Wellengeneratoren sind mit 7 bzw. 8 bezeichnet. Die Leuchtdioden 5 und 6 sind auf einem Träger 4 angeordnet, der beispielsweise aus einer Leiterplatte bestehen kann. Die Leuchtdioden 5 und 6 sind von einer Vergußmasse 11 umgeben, die vorzugsweise aus einem transparenten Kunststoff bestehen kann. Die rote Strahlung 13 und die infrarote Strahlung 14 durchdringen das Meßmedium 9, das einen Meßkanal 12 mit einem Querschnitt von vorzugsweise weniger als 1 mm durchströmt, der in einen transparenten Kanalkörper 15 eingefräst ist, der vorzugsweise aus Polymethylmethacrylat (Plexiglas) bestehen kann. Dieser Kanalkörper 15 ist auf die Vergußmasse 11 flüssigkeitsdicht aufgesetzt und kann vorzugsweise noch mit in der Figur nicht näher bezeichneten Dichtungen versehen sein. In die Stirnfläche des Kanalkörpers 15 ist ein Strahlungsempfänger 16 eingesetzt, der beispielsweise aus einer Halbleiterdiode, vorzugsweise einer Siliziumphotodiode, bestehen kann, deren Anode in der Figur mit 17 und deren Kathode mit 18 bezeichnet ist. Zur Demodulation der Strahlungen 13 und 14 ist eine Elektronik 20 vorgesehen, die einen Stromspannungswandler 22 und zwei Lock-in-Verstärker 24 und 25 enthält, von denen der Lock-in-Verstärker 24 für die rote Strahlung und der Lock-in-Verstärker 25 für die infrarote Strahlung vorgesehen sein soll. Die entsprechenden Ausgänge sind in der Figur mit 26 bzw. 27 bezeichnet.

Mit der Elektronik 20 werden die Intensitäten der durch den Meßkanal 12 geschwächten roten Strahlung 13 und infraroten Strahlung 14 bestimmt. Da ein Abdunkeln der Meßzelle 10 nur mit verhältnismäßig großem Aufwand möglich ist, wird durch die Elektronik 20 eine Trennung von Hintergrund- und Nutzstrahlung gewährleistet.

In einer besonderen Ausführungsform der Anordnung wird der infraroten Leuchtdiode 6 ein Gleichstrom von beispielsweise $I_{g1}$ = 3,2 mA aufgeprägt und mit einem Wechselstrom von beispielsweise $I_{eff}$ = 1,2 mA bei einer Trägerfrequenz von beispielsweise $f_1$ = 700 Hz moduliert. Die rote Leuchtdiode 5 wird beispielsweise mit $I_{g1}$ = 27 mA und $I_{eff}$ = 9,2 mA und einer Trägerfrequenz $f_2$ = 570 Hz betrieben. Mit dieser unterschiedlichen Betriebsweise erhält man mit der spektralen Empfindlichkeit des Strahlungsempfängers 16 und der unterschiedlichen Leuchtstärke der Leuchtdioden 5 und 6 ein wenigstens annähernd gleiches Intensitätssignal. Der Photostrom an der Kathode 18 des Strahlungsempfängers 16 wird in einem vorzugsweise mehrstufigen, insbesondere dreistufigen, Eingangsverstärker in ein Spannungssignal umgewandelt. Der Gleichstromanteil wird abgekoppelt und auf eine effektive Spannung von beispielsweise $U_{eff}$ = 2 V verstärkt. Das Ausgangssignal wird auf die beiden Lock-in-Verstärker 24 und 25 gegeben und entsprechend den beiden Trägerfrequenzen $f_1$ und $f_2$ für die infrarote und rote Strahlung demoduliert. Nach einer in der Figur nicht dargestellten ausgangsseitigen Tiefpaßfilterung erhält man ein der roten Strahlung 13 bzw. infraroten Strahlung 14 proportionales Ausgangssignal an den Ausgängen 26 bzw. 27.

Gemäß Figur 2 ist der Meßzelle 10 mit ihrer Elektronik 20 ein Probenahmesystem zugeordnet, das abwechselnd das Meßmedium 9 und die Infusionslösung 39 durch den Meßkanal 12 strömen läßt. In diesem Probenahmesystem verbindet der Meßkanal 12 eine Vorrichtung 28 zur Konstanthaltung der Strömungsgeschwindigkeit im Meßkanal 12 mit einem Lumen 29 eines Mehrlumenkatheters mit wenigstens zwei Lumen, der aber zur Vereinfachung als Doppellumenkatheter 30 bezeichnet werden soll. Die Öffnung des inneren Lumens 31 ist in der Figur mit 33 bezeichnet. Das zweite Lumen 32 ist über eine umsteuerbare Pumpe 36 mit einem Behälter 38 für eine Infusionslösung 39 verbunden. Die beiden Lumen 31 und 32 haben eine gemeinsame Mündung 34.

Die Vorrichtung 28 kann vorzugsweise aus einer Schlauchpumpe bestehen, die eine konstante Meßströmung gewährleistet. Ferner ist beispielsweise auch ein Drosselventil geeignet. Die Meßzelle 10, deren gesamte Ausdehnung im allgemeinen 10 mm nicht wesentlich überschreitet und insbesondere weniger als 10 mm betragen kann und die vorzugsweise mit dem Doppellumenkatheter 30 eine gemeinsame Baueinheit bildet, stellt eine sehr kompakte Bauform der Anordnung dar, die im allgemeinen von der Vorrichtung 28 und der Elektronik 20 räumlich getrennt ist.

Zur Inbetriebnahme der Anordnung zur Untersuchung des flüssigen Meßmediums 9 wird zunächst die Infusionslösung 39, vorzugsweise eine physiologische Elektrolytlösung, insbesondere eine Ringer-Lösung, mit Hilfe der Pumpe 36 durch einen Infusionsschlauch 42 in das äußere Lumen 32 des Doppellumenkatheters 30 bis in das Meßmedium 9, nämlich die Blutströmung der Arterie 40, des Patienten gepumpt. Zugleich wird durch die Vorrichtung 28 über die Öffnung 33 des inneren Lumens 31 ein Teil dieser Infusionslösung 39 durch den Meßkanal 12 der Meßzelle 10 gezogen. Mit der Umkehrung der Strömung an der gemeinsamen Mündung 3 4, beispielsweise durch Umsteuerung der Pumpe 36, wird an der Mündung 33 des inneren Lumens 31 Blut aus der Arterie 40 gezogen, das in das innere Lumen 31 einströmt. Sobald eine vorbestimmte Menge Blut in den Meßkanal 12 eingeströmt ist, wird die Pumpe 36 wieder umgepolt. Im Rhythmus der Umsteuerung der Pumpe 36 wird von der Vorrichtung 28 eine Folge von abwechselnd Meßmedium 9 und der Infusionslösung 39 durch den Meßkanal 12 der Meßzelle 10 gezogen, deren Gesamtmenge für einen Meßvorgang 20 µl im allgemeinen nicht wesentlich überschreitet.

Eine weitere Ausführungsform der Anordnung zur Untersuchung von Vollblut als Meßmedium 9 besteht darin, daß dem Meßkanal 12 eine Vorrichtung zur Hämolyse zugeordnet ist, die bewirkt, daß das durch den Meßkanal 12 strömende Vollblut vor der Messung vollständig hämolysiert wird. Zu diesem Zweck kann das Blut im Meßkanal 12 vorzugsweise einem Ultraschallfeld ausgesetzt werden. Der entsprechende Ultraschallsender ist in Figur 2 mit 48 bezeichnet.

Im Ausführungsbeispiel gemäß Figur 2 ist ein Doppellumenkathe ter 30 dargestellt, bei dem ein Lumen 31 von dem anderen 32 konzentrisch umgeben ist. Es können jedoch beispielsweise auch zwei oder mehrere getrennte Lumen mit einer gemeinsamen Öffnung zum Meßmedium nebeneinander angeordnet sein. Ferner können um das äußere Lumen 32 noch weitere Lumen konzentrisch angeordnet sein, die alle mit der gemeinsamen Öffnung 34 versehen sind.

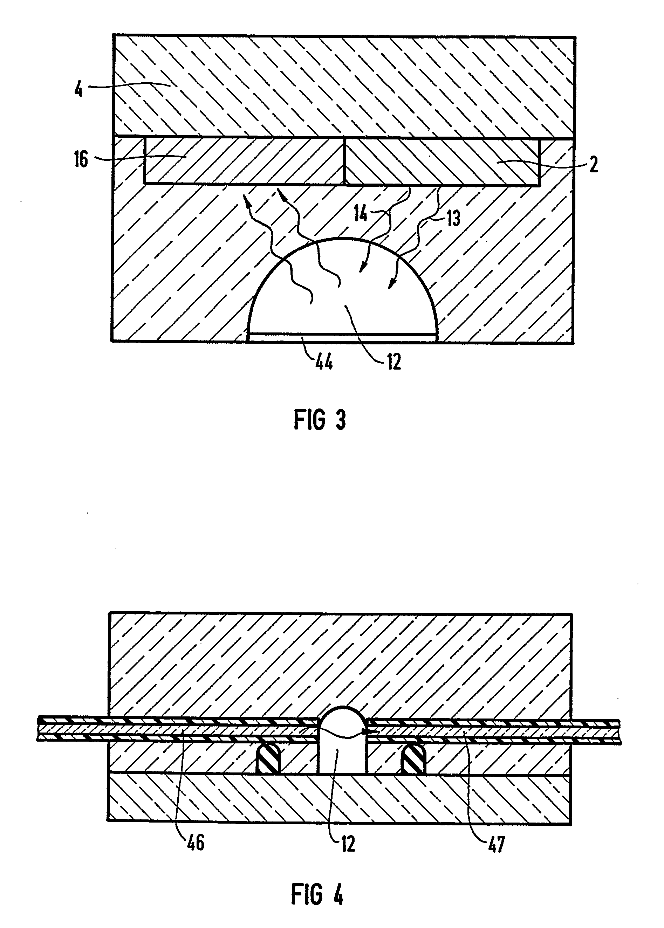

In einer besonderen Ausführungsform der Meßzelle 10 gemäß Figur 3 können die Strahlungsquelle 2 und der Strahlungsempfänger 16 auf der gleichen Seite des Meßkanals 12 angeordnet werden, vorzugsweise mit einem Halbleiterkörper, insbesondere aus Silizium, ein gemeinsames Halbleiterbauelement bilden, das auf dem Träger 4 angeordnet ist. Zur Reflexion der Strahlungen 13 und 14 kann dann beispielsweise ein Spiegel 44 auf der gegenüberliegenden Seite des Meßkanals 12 derart angeordnet sein, daß die reflektierte Strahlung auf den Empfänger 16 fällt.

In der Ausführungsform gemäß Figur 4 sind die nicht dargestellte Strahlungsquelle sowie der Strahlungsempfänger außerhalb der Meßzelle angeordnet und die Strahlungen werden mit einem Lichtleiter 46 dem Meßkanal 12 und mit einem weiteren Lichtleiter 47 dem Strahlungsempfänger zugeführt.

In der Ausführungsform der Anordnung zur Bestimmung der Konzentration eines Indikatorfarbstoffs, beispielsweise Indocyaningrün, im Meßmedium 9 ist eine Strahlungsquelle 2 für noch eine dritte Wellenlänge, beispielsweise $\lambda_3$ = 800 nm, vorgesehen und die Elektronik 20 ist dementsprechend zur Demodulation der drei Strahlungen ausgerüstet. Damit ist beispielsweise eine Bestimmung des Herz-Zeitvolumens möglich, bei welcher die Farbstoffänderung in Abhängigkeit von der Zeit ermittelt wird.

In einer Ausführungsform der Anordnung, bei der hämolysiertes Vollblut zur Messung vorgesehen ist, kann unter Verwendung von mehr als 2 Wellenlängen auch zusätzlich der Gehalt des Vollblutes an Carboxyhämoglobin und Methämoglobin bestimmt werden.

Im Ausführungsbeispiel ist die Verwendung der Anordnung zur Untersuchung eines flüssigen Meßmediums zur Blutuntersuchung vorgesehen. Die Anordnung kann jedoch auch auf anderen technischen Sachgebieten vorteilhaft angewendet werden, beispielsweise in der Biotechnologie zur Prozeßkontrolle und Prozeßsteuerung in einem Bioreaktor.

## Ansprüche

1. Anordnung zur Untersuchung eines flüssigen Meßmediums mit einer Meßzelle, die das Meßmedium enthält und der eine Elektronik zugeordnet ist, **gekennzeichnet** durch folgende Merkmale:

a) der Meßkanal (12) wird abwechselnd vom Meßmedium (9) und von einer Infusionslösung (39) durchströmt und durchsetzt die Meßzelle (10),

b) diese Meßzelle (10) enthält eine Strahlungsquelle (2) für mindestens zwei Wellenlängen ($\lambda_1$, $\lambda_2$), deren Strahlungen (13 bzw. 14) den Meßkanal (12) durchdringen und von einem Strahlungsempfänger (16) aufgenommen werden,

c) der Meßkanal (12) verbindet eine Vorrichtung (28) zur Konstanthaltung der Strömungsgeschwindigkeit des Meßmediums (9) im Meßkanal (12) mit einem Lumen (31) eines Doppellumenkatheters (30),

d) dem zweiten Lumen (32) des Doppellumenkatheters (30) ist die Infusionslösung (39) mit umkehrbarer Strömungsrichtung zuführbar,

e) beide Lumen (31, 32) haben eine gemeinsame Mündung (34), welche in das Meßmedium (9) einführbar ist (Figuren 1 und 2).

2. Anordnung nach Anspruch 1, **dadurch gekennzeichnet,** daß die Strahlungsquelle (2) und der Strahlungsempfänger (16) auf der gleichen Seite des Meßkanals (12) angeordnet sind und zur Reflexion der Strahlungen (13, 14) ein Spiegel (44) vorgesehen ist.

3. Anordnung nach Anspruch 2, **dadurch gekennzeichnet,** daß die Strahlungsquelle (2) und der Strahlungsempfänger (16) ein gemeinsames Halbleiterbauelement bilden (Figur 3).

4. Anordnung zur Untersuchung eines flüssigen Meßmediums, das in einem Meßkanal angeordnet ist, dem eine Strahlungsquelle und ein Strahlungsempfänger zugeordnet sind, **gekennzeichnet** durch folgende Merkmale:

a) der Meßkanal (12) wird abwechselnd vom Meßmedium (9) und von einer Infusionslösung (39) durchströmt,

b) der Meßkanal (12) verbindet eine Vorrichtung (28) zur Konstanthaltung der Strömungsgeschwindigkeit des Meßmediums (9) im Meßkanal (12) mit einem Lumen (31) eines Doppellumenkatheters (30),

c) dem zweiten Lumen (32) des Doppellumenkatheters (30) ist die Infusionslösung mit umkehrbarer Strömungsrichtung zuführbar,

d) beide Lumen (31, 32) haben eine gemeinsame Mündung (34), welche in das Meßmedium einführbar ist,

d) zur Zuführung der Strahlungen (13, 14) über den Meßkanal (12) zum Strahlungsempfänger (16) sind Lichtleiter (46, 47) vorgesehen (Figur 4).

5. Anordnung nach Anspruch 1 zur Untersuchung von Vollblut, **dadurch gekennzeichnet,** daß dem Meßkanal (12) eine Vorrichtung zur Hämolyse zugeordnet ist.

6. Anordnung nach Anspruch 5, **dadurch gekennzeichnet,** daß eine Teilstrecke des Meßkanals (12) einem Ultraschallfeld ausgesetzt ist.

7. Anordnung nach Anspruch 1 zur Bestimmung einer Farbstoffkonzentration im Meßmedium, **dadurch gekennzeichnet,** daß eine Strahlungsquelle für mindestens drei Wellenlängen vorgesehen ist.

FIG 1

FIG. 2

FIG 3

FIG 4

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| D,Y | EP-A-273258 (SIEMENS AKTIENGESELLSCHAFT) <br> * Zusammenfassung * <br> * Spalte 4, Zeile 23 - Spalte 7, Zeile 7 * <br> --- | 1, 4 | A61B5/00 |
| Y | US-A-4447150 (HEINEMANN) <br> * Spalte 4, Zeilen 15 - 45 * <br> * Spalte 6, Zeile 38 - Spalte 10, Zeile 28 * <br> * Spalte 15, Zeilen 44 - 51 * <br> * Figuren 2a-5, 7 * | 1, 4 | |
| A | | 2, 3 | |
| | --- | | |
| D,A | MEDIZINTECHNIK. <br> vol. 107, no. 3, 1987, ZUERICH CH <br> Seiten 107 - 108; Schubert: <br> "Blutgas-Messgeräte (in vivo)" <br> * das ganze Dokument * <br> --- | 1, 4, 7 | |
| A | EP-A-276535 (MITSUBISHI RAYON CO. LTD.) <br> * Spalte 5, Zeile 42 - Spalte 7, Zeile 31 * <br> * Spalte 9, Zeile 32 - Spalte 10, Zeile 30 * <br> * Figuren * <br> --- | 1, 4 | RECHERCHIERTE SACHGEBIETE (Int. Cl.5 ) |
| A | EP-A-10577 (CONTRAVES) <br> * Zusammenfassung * <br> ----- | 5 | A61B <br> G01N |

Der vorliegende Recherchenbericht wurde für alle Patentansprüche erstellt

| Recherchenort | Abschlußdatum der Recherche | Prüfer |
|---|---|---|
| DEN HAAG | 16 FEBRUAR 1990 | CHEN A.H. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
..........................................................................
& : Mitglied der gleichen Patentfamilie, übereinstimmendes
    Dokument

EPO FORM 1503 03.82 (P0403)